# FASCICULE DE BREVET EUROPEEN

(11) **EP 2 881 074 B1**
(45) Date de publication et mention de la délivrance du brevet: **18.04.2018**
(21) Numéro de dépôt: 13196222.7
(22) Date de dépôt: 09.12.2013
(51) Int. Cl.: A61C 1/05, A61C 1/12, A61B 17/16

(54) **Pièce à main dentaire ou chirurgicale à air comprimé et turbine pour une telle pièce à main**
Druckluftbetriebenes zahnchirurgisches oder chirurgisches Handstück und Turbine für ein solches Handstück
Dental or surgical compressed-air handpiece and turbine for such a handpiece

(43) Date de publication de la demande: 10.06.2015
(60) Demande divisionnaire: 18158188.5
(73) Titulaire: Bien-Air Holding SA, 2500 Bienne 6 (CH)
(72) Inventeur: Farine, Laurent, 2740 Moutier (CH)
(74) Mandataire: Supper, Marc

(56) Documents cités:
- US-A- 3 893 242
- US-A- 4 320 927
- US-A- 5 286 194
- US-A1- 2011 281 232

## Description

La présente invention concerne une pièce à main dentaire ou chirurgicale à air comprimé ainsi qu'une turbine pour une telle pièce à main. La présente invention concerne en particulier une pièce à main à air comprimé présentant une puissance accrue.

La présente invention concerne le domaine des pièces à main à usage chirurgical ou dentaire. Il existe deux familles de pièces à main : les contre-angles qui sont équipés d'un moteur électrique et les pièces à main qui comprennent une turbine entraînée par de l'air comprimé.

On s'intéresse ici aux pièces à main à air comprimé. Très schématiquement, ces pièces à main à air comprimé comprennent une tête à l'intérieur de laquelle est logée une turbine. Cette turbine comprend un conduit d'amenée de l'air comprimé et une roue mobile munie sur sa périphérie d'une pluralité d'aubes régulièrement espacées. Le conduit d'amenée sert à forcer sur les aubes de la turbine un flux d'air comprimé dont l'énergie pneumatique se transforme en énergie cinétique lorsque le flux d'air comprimé frappe les aubes de la roue mobile.

La figure 1 annexée à la présente demande de brevet est une vue en perspective et en coupe selon un plan horizontal coupant longitudinalement le manche et la tête d'une pièce à main à air comprimé selon l'art antérieur. Désignée dans son ensemble par la référence numérique générale 1, cette pièce à main comprend un manche 2 qui se raccorde à une tête 4 à l'intérieur de laquelle est logée une turbine 6. La turbine 6 comprend une roue mobile 8 depuis une périphérie 10 de laquelle s'étend une pluralité d'aubes 12 qui définissent un diamètre extérieur 14 de la roue mobile 8.

La pièce à main 1 comprend également des moyens d'injection ayant pour fonction de diriger sur les aubes 12 de la roue mobile 8 un flux d'air comprimé dont l'énergie pneumatique se transforme en énergie cinétique lorsque le flux d'air comprimé frappe les aubes 12 de la roue mobile 8. Les moyens d'injection de l'air comprimé se composent d'un conduit 16 d'amenée de l'air comprimé qui est usiné selon des techniques conventionnelles dans le manche 2 de la pièce à main 1. Par ailleurs, un conduit 18 d'échappement de l'air est également usiné par des techniques classiques dans le manche 2 de la pièce à main 1, sensiblement parallèlement et à distance du conduit 16 d'amenée de l'air comprimé.

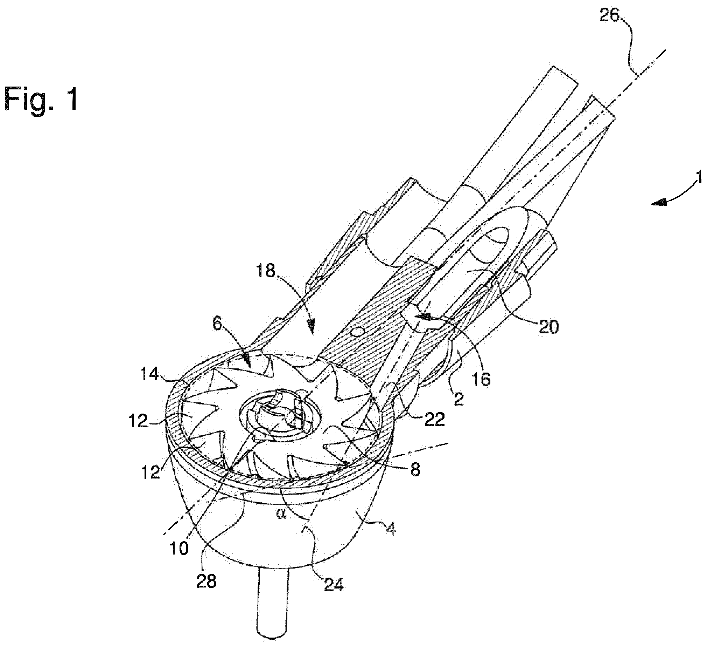

On voit, à l'examen de la figure 1, que le conduit 16 d'amenée de l'air comprimé se compose d'un premier et d'un second conduit rectilignes, respectivement 20 et 22, disposés dans le prolongement l'un de l'autre, l'axe longitudinal de symétrie 24 du second conduit rectiligne 22 s'étendant légèrement de biais par rapport à l'axe longitudinal de symétrie 26 du manche 2 de la pièce à main 1 et formant un angle α non-nul avec la tangente 28 au diamètre extérieur 14 de la roue mobile 8. On comprend qu'il est difficilement envisageable d'usiner le conduit 16 d'amenée de l'air comprimé selon une direction qui tendrait à se rapprocher de la tangente 28 au diamètre extérieur 14 de la roue mobile 8, au risque de percer le conduit 18 d'échappement de l'air ou de devoir réduire le diamètre de ce conduit 18. Le document US 5,286,194 A divulgue une pièce à main selon le préambule de la revendication 1. Un problème récurrent qui se pose aux constructeurs de pièces à main à air comprimé réside dans l'efficacité de la conversion entre l'énergie pneumatique de l'air comprimé et l'énergie cinétique de la roue mobile de la turbine. En effet, de l'efficacité de cette conversion dépend la puissance mécanique que peut fournir la pièce à main à air comprimé. Cette efficacité de conversion est notamment étroitement liée à la chute de pression entre la pression à laquelle se trouve l'air comprimé qui pénètre dans la tête de la pièce à main, et la pression de l'air qui s'échappe de la tête de la pièce à main après que l'air comprimé a frappé les aubes de la turbine. En effet, plus cette chute de pression est importante, meilleure est la conversion entre l'énergie pneumatique et l'énergie cinétique. L'efficacité de conversion entre l'énergie pneumatique et l'énergie cinétique est également d'autant meilleure que l'écoulement de l'air se fait avec le moins de turbulences et donc le moins de pertes possible. On cherche également à réduire les bruits de fonctionnement des pièces à main à air comprimé et à éviter autant que possible que l'air comprimé destiné à actionner la turbine ne fuie dans la bouche du patient

La présente invention a pour but de répondre aux objectifs mentionnés ci-dessus ainsi qu'à d'autres encore en procurant notamment une pièce à main fournissant une puissance mécanique plus élevée.

A cet effet, la présente invention concerne une pièce à main à air comprimé selon la revendication 1. Elle comprend un manche qui se raccorde à une tête à l'intérieur de laquelle est logée une turbine, la turbine comprenant une roue mobile depuis une périphérie de laquelle s'étendent une pluralité d'aubes qui définissent un diamètre extérieur de la roue mobile, la pièce à main comprenant également des moyens d'injection ayant pour fonction de diriger sur les aubes de la roue mobile un flux d'air comprimé dont l'énergie pneumatique se transforme en énergie cinétique lorsque le flux d'air comprimé frappe les aubes de la roue mobile, la pièce à main comprenant de plus un conduit d'échappement ménagé dans le manche de la pièce à main et par lequel s'échappe l'air après avoir frappé les aubes de la roue mobile, la pièce à main étant caractérisée en ce que le conduit d'échappement se trouve au moins en regard de la zone dans laquelle l'air comprimé frappe les aubes de la roue mobile.

Selon une caractéristique de l'invention, la section du conduit d'échappement s'étend sur au moins la hauteur des aubes de la roue mobile.

Selon encore une autre caractéristique de l'invention, les moyens d'injection de l'air comprimé se présentent sous la forme d'un insert à l'intérieur duquel est ménagé un conduit d'amenée du flux d'air comprimé et qui est rapporté à l'intérieur du manche de la pièce à main.

Selon encore une autre caractéristique de l'invention, l'insert est disposé à l'intérieur du conduit d'échappement.

Selon encore une autre caractéristique de l'invention, l'insert est monté de manière coaxiale à l'intérieur du conduit d'échappement.

Selon un mode préféré de réalisation de l'invention, l'insert est agencé de façon que l'axe central du flux d'air comprimé s'étende selon une direction tangentielle au diamètre extérieur de la roue mobile.

Selon encore un mode préféré de réalisation de l'invention, le conduit d'amenée du flux d'air comprimé se compose d'un premier et d'un second conduit rectilignes disposés dans le prolongement l'un de l'autre, le second conduit rectiligne présentant un axe longitudinal de symétrie qui s'étend selon une direction tangentielle au diamètre extérieur de la roue mobile.

Grâce à ces caractéristiques, la présente invention procure une pièce à main dans laquelle les moyens d'injection se présentent sous la forme d'un insert destiné à être monté à l'intérieur du manche de la pièce à main. Le fait de disposer d'une pièce rapportée permet de canaliser le flux d'air selon une direction tangente au diamètre extérieur de la roue mobile plus facilement que dans le cas où le conduit d'amenée de l'air est usiné par des techniques classiques dans le corps de la pièce à main. En effet, dans les pièces à main à air comprimé, la turbine est agencée dans une tête qui est typiquement disposée dans le prolongement d'un manche de préhension généralement rectiligne à l'intérieur duquel est usiné le conduit d'amenée de l'air comprimé. Sauf à prendre des mesures complexes et donc économiquement peu viables, il n'est pas possible d'usiner des portions coudées par des techniques classiques dans le manche de préhension de la pièce à main. Par conséquent, le conduit d'amenée de l'air comprimé doit être usiné de biais par rapport à l'axe longitudinal de symétrie du manche de préhension si l'on souhaite que le flux d'air vienne frapper les aubes de la turbine selon une direction qui se rapproche de la tangente au diamètre extérieur de la roue mobile. Or, il faut également pouvoir usiner dans le manche de préhension de la pièce à main un conduit d'échappement par lequel s'échappe l'air après avoir frappé les aubes de la roue mobile. L'inclinaison du conduit d'amenée de l'air comprimé par rapport à l'axe longitudinal de symétrie du manche de préhension est donc nécessairement limitée car, dans le cas contraire, le conduit d'amenée de l'air comprimé déboucherait dans le conduit d'échappement, ce qui est techniquement inenvisageable.

Selon un autre avantage de l'invention, l'insert qui sert à forcer le flux d'air comprimé en direction des aubes de la roue mobile est monté à l'intérieur du conduit d'échappement ménagé dans le corps de la pièce à main et par lequel s'échappe l'air après avoir frappé les aubes de la roue mobile. Un tel agencement facilite le retour d'air et favorise donc grandement la chute de pression entre la pression de l'air comprimé avant qu'il ne frappe les aubes de la turbine et la pression de l'air lorsqu'il s'échappe de la tête de la pièce à main après avoir frappé les aubes de la turbine. Or, plus cette chute de pression est importante, meilleure est la conversion entre l'énergie pneumatique de l'air comprimé et l'énergie cinétique de la turbine. Cette chute de pression est encore amplifiée par le fait que l'insert est monté de manière coaxiale à l'intérieur du conduit d'échappement. Le diamètre du conduit d'échappement peut donc être augmenté, ce qui permet de ramener la pression de l'air dans la tête de la pièce à main à des valeurs proches de la pression atmosphérique. Enfin, l'efficacité de conversion entre l'énergie pneumatique et l'énergie cinétique est encore améliorée du fait que l'écoulement de l'air se fait avec le moins de turbulences et donc le moins de pertes possibles. D'autre part, les bruits de fonctionnement de la pièce à main sont réduits et on évite pratiquement toute fuite d'air dans la bouche du patient.

Selon un autre avantage de l'invention, les moyens d'injection forcent le flux d'air comprimé de façon que le flux d'air comprimé vienne frapper les aubes de la roue mobile de la turbine selon une direction tangente au diamètre extérieur de la roue mobile. De la sorte, on garantit que le couple exercé par le flux d'air sur les aubes de la turbine est maximum, ce qui permet d'accroître sensiblement la puissance délivrée par la turbine. A titre d'exemple, la puissance délivrée par les pièces à main à air comprimé actuelles est de l'ordre de 13 watts, alors que la puissance mesurée d'une pièce à main à air comprimé selon l'invention est de l'ordre de 20 watts. Les praticiens disposent donc avec une pièce à main à air comprimé selon l'invention d'une puissance accrue, ce qui leur permet de travailler plus rapidement ou d'effectuer des soins qui, jusqu'à présent, pouvaient être difficilement réalisés à l'aide d'une pièce à main à air comprimé en raison de la trop faible puissance disponible.

Selon un mode préféré de réalisation de l'invention, la première surface s'étend depuis le diamètre extérieur de la roue mobile jusqu'à un point du rayon de la roue mobile à partir duquel s'étend radialement la seconde surface jusqu'au diamètre extérieur de la roue mobile.

Enfin, selon une autre caractéristique de l'invention, les aubes sont associées par paire de manière étagée, chaque aube étant séparée de son aube correspondante par une échancrure qui s'étend dans la direction du flux d'air comprimé, de sorte que le flux d'air se partage en deux parts égales au moment où il atteint l'échancrure. Cette échancrure a pour but de réduire le temps de passage d'une aube à l'aube suivante afin de limiter les pertes de puissance mécanique de la turbine.

La première surface conduit vers la seconde surface l'air comprimé qui sort des moyens d'injection. Comme la seconde surface s'étend selon un rayon de la roue mobile et que le flux d'air comprimé est canalisé selon une direction tangente au diamètre extérieur de la roue mobile, le flux d'air comprimé tombe perpendiculairement sur la seconde surface de l'aube puis fait un demi-tour. Or, la puissance mécanique délivrée par une turbine est d'autant plus grande que l'angle de déviation de l'air comprimé est important. Dans le cas de la présente invention, l'angle de déviation de l'air comprimé est de 180°, ce qui correspond à un optimum de conversion entre l'énergie pneumatique de l'air comprimé et l'énergie cinétique de la turbine.

D'autres caractéristiques et avantages de la présente invention ressortiront plus clairement de la description détaillée qui suit d'un mode de réalisation d'une pièce à main à air comprimé selon l'invention, cet exemple étant donné à titre purement illustratif et non limitatif seulement en liaison avec le dessin annexé sur lequel :
- la figure 1, déjà mentionnée, est une vue en perspective et en coupe selon un plan horizontal coupant longitudinalement le manche et la tête d'une pièce à main à air comprimé selon l'art antérieur ;
- la figure 2 est une vue en coupe selon un plan horizontal coupant longitudinalement le manche et la tête d'une pièce à main à air comprimé selon l'invention ;
- la figure 3 est une vue de côté, partiellement en transparence, de la tête et du manche de la pièce à main à air comprimé selon l'invention ;
- la figure 4 est une représentation schématique illustrant les première et seconde surfaces d'une aube de la turbine ;
- la figure 5 illustre une variante de réalisation perfectionnée de la turbine selon l'invention ;
- la figure 6 est une vue de l'arrière de la tête de la pièce à main à air comprimé selon l'invention, dans la zone où la tête de la pièce à main se raccorde au manche de la pièce à main ;
- la figure 7 est une vue en coupe selon un plan vertical passant par l'axe longitudinal de symétrie de la tête et d'une portion du manche de la pièce à main à air comprimé selon l'invention, et
- la figure 8 est une représentation schématique d'une variante de réalisation de la pièce à main à air comprimé sortant du cadre de l'invention.

La présente invention procède de l'idée générale inventive qui consiste à prendre toutes les mesures nécessaires pour accroître la puissance mécanique que peut délivrer une pièce à main à air comprimé. A cet effet, la présente invention cherche à optimiser l'efficacité de la conversion entre l'énergie pneumatique de l'air comprimé et l'énergie cinétique de la roue mobile de la turbine. En effet, de l'efficacité de cette conversion dépend la puissance mécanique que peut fournir la pièce à main à air comprimé. Cette efficacité de conversion est notamment étroitement liée aux conditions d'écoulement de l'air dans la tête de la turbine. Effectivement, on sait que tandis qu'une partie de l'air comprimé injecté dans la tête de la pièce à main contourne la turbine en entraînant celle-ci, une autre partie de l'air comprimé ne fait que ricocher sur les aubes de la turbine et tend donc à revenir en arrière en créant des perturbations dans la tête de la pièce à main. En prévoyant d'agencer les moyens d'échappement de l'air au moins dans la zone où cet air est injecté, la présente invention facilite l'évacuation de l'air comprimé qui ricoche sur les aubes de la turbine et qui tend à créer des turbulences dans la tête de la pièce à main. L'efficacité de conversion entre l'énergie pneumatique et l'énergie cinétique est également liée à l'angle selon lequel l'air comprimé frappe les aubes de la turbine. C'est pourquoi, selon un premier aspect, la présente invention enseigne de réaliser les moyens d'amenée de l'air comprimé sous la forme d'un insert rapporté dans la pièce à main. Le fait de disposer d'une pièce rapportée permet au constructeur de jouir d'une liberté dans le positionnement de l'insert plus grande que dans le cas où le constructeur devrait usiner un conduit d'amenée de l'air comprimé par des techniques classiques dans le manche de la pièce à main. Selon un autre aspect de l'invention, l'insert d'amenée de l'air comprimé est placé de manière coaxiale dans le conduit d'échappement de l'air comprimé. Cette disposition limite les turbulences et facilite donc le retour d'air, ce qui favorise la chute de pression entre la pression de l'air comprimé avant qu'il ne frappe les aubes de la turbine et la pression de l'air lorsqu'il s'échappe de la tête de la pièce à main après avoir frappé les aubes de la turbine. L'efficacité de la conversion entre l'énergie pneumatique de l'air comprimé et l'énergie cinétique de la roue mobile de la turbine est ainsi également optimisée. Pour atteindre ce résultat, la présente invention enseigne également de canaliser le flux d'air comprimé selon une direction tangente au diamètre extérieur de la turbine de la pièce à main à air comprimé. De la sorte, le flux d'air comprimé frappe les aubes de la turbine perpendiculairement à leur surface, ce qui permet de garantir que le couple exercé par le flux d'air sur les aubes de la turbine est maximum et donc d'accroître sensiblement la puissance délivrée par la turbine. Enfin, les aubes de la turbine se composent chacune d'une première surface qui s'étend depuis le diamètre extérieur de la roue mobile et mène jusqu'à une seconde surface qui s'étend selon un rayon de la roue mobile en s'éloignant du centre de la roue mobile. L'air comprimé tombe donc perpendiculairement sur la seconde surface de l'aube et est refoulé vers l'arrière en étant dévié de 180°. Or, plus l'angle de déviation du flux d'air comprimé est important, plus la force exercée sur les aubes de la turbine est importante. Enfin, les bruits de fonctionnement de la turbine selon l'invention sont limités et l'on travaille quasiment à la pression atmosphérique, de sorte que très peu d'air fuie dans la bouche du patient.

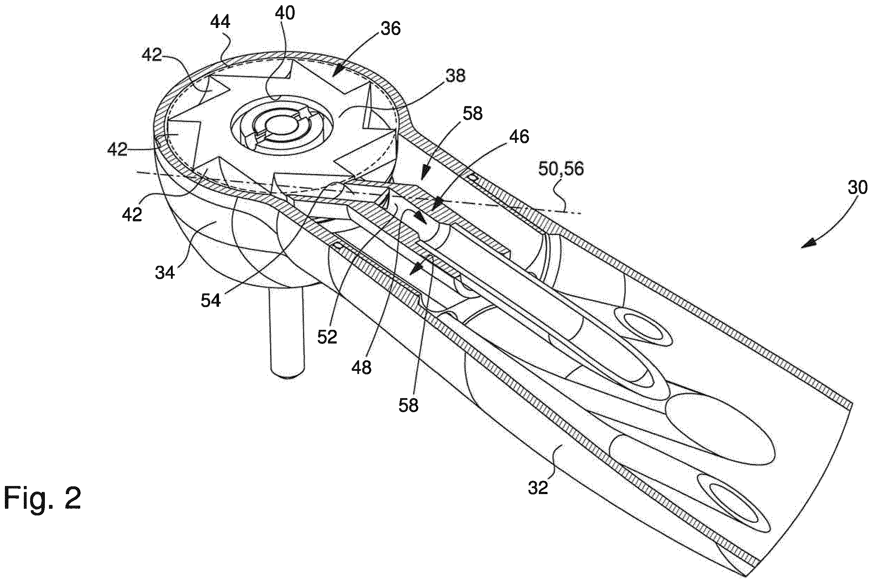

La figure 2 est une vue en coupe selon un plan horizontal coupant longitudinalement le manche et la tête d'une pièce à main à air comprimé selon l'invention. Désignée dans son ensemble par la référence numérique générale 30, la pièce à main selon l'invention comprend un manche 32 qui se raccorde à une tête 34 à l'intérieur de laquelle est logée une turbine 36. La turbine 36 comprend une roue mobile 38 depuis une périphérie 40 de laquelle s'étend une pluralité d'aubes 42 qui définissent un diamètre extérieur 44 de la roue mobile 38.

La pièce à main 30 comprend également des moyens d'injection ayant pour fonction de diriger sur les aubes 42 de la roue mobile 38 un flux d'air comprimé dont l'énergie pneumatique se transforme en énergie cinétique lorsque le flux d'air comprimé frappe les aubes 42 de la roue mobile 38.

A cet effet, les moyens d'injection de l'air comprimé se présentent sous la forme d'un insert 46 indépendant monté à l'intérieur de la pièce à main 30 et à l'intérieur duquel est ménagé un conduit 48 d'amenée du flux d'air comprimé. De manière préférée mais non limitative, le conduit 48 est agencé de façon que l'axe central 50 du flux d'air comprimé s'étende selon une direction tangentielle au diamètre extérieur 44 de la roue mobile 38. La présente invention procure ainsi une pièce à main 30 dans laquelle les moyens d'injection forcent le flux d'air comprimé de façon que le flux d'air comprimé vienne frapper les aubes 42 de la roue mobile 38 de la turbine 36 selon une direction tangente au diamètre extérieur 44 de la roue mobile 38. De la sorte, on garantit que le couple exercé par le flux d'air sur les aubes 42 de la turbine 36 est maximum, ce qui permet d'accroître sensiblement la puissance délivrée par la turbine 36.

Selon une forme préférée mais non limitative de réalisation de l'invention, le conduit 48 d'amenée du flux d'air comprimé se compose d'un premier et d'un second conduit rectilignes, respectivement 52 et 54, disposés dans le prolongement l'un de l'autre, l'axe longitudinal de symétrie 56 du second conduit rectiligne 54 s'étendant selon une direction tangentielle au diamètre extérieur 44 de la roue mobile 38.

Selon encore une autre caractéristique de l'invention, l'insert 46 qui sert à forcer le flux d'air comprimé en direction des aubes 42 de la roue mobile 38 est monté à l'intérieur d'un conduit d'échappement 58 ménagé dans le manche 32 de la pièce à main 30 et par lequel s'échappe l'air après avoir frappé les aubes 42 de la roue mobile 38. De manière préférée mais non limitative, l'insert 46 est monté de manière coaxiale à l'intérieur du conduit d'échappement 58.

L'agencement ci-dessus favorise grandement la chute de pression entre la pression de l'air comprimé avant qu'il ne frappe les aubes 42 de la turbine 36 et la pression de l'air lorsqu'il s'échappe de la tête 34 de la pièce à main 30 après avoir frappé les aubes 42 de la turbine 36. Or, plus cette chute de pression est importante, meilleure est la conversion entre l'énergie pneumatique de l'air comprimé et l'énergie cinétique de la turbine 36. Cette chute de pression est encore amplifiée par le fait que l'insert 46 est monté de manière coaxiale à l'intérieur du conduit d'échappement 58. Le diamètre du conduit d'échappement 58 peut donc être augmenté, ce qui facilite le retour d'air et permet de ramener la pression de l'air dans la tête 34 de la pièce à main 30 à des valeurs proches de la pression atmosphérique. Enfin, l'efficacité de conversion entre l'énergie pneumatique et l'énergie cinétique est encore améliorée du fait que l'écoulement de l'air se fait avec le moins de turbulences et donc le moins de pertes possibles. D'autre part, les bruits de fonctionnement de la pièce à main 30 sont réduits et on évite pratiquement toute fuite d'air dans la bouche du patient dans la mesure où la pression dans la tête 34 de la turbine 36 est proche de la pression atmosphérique.

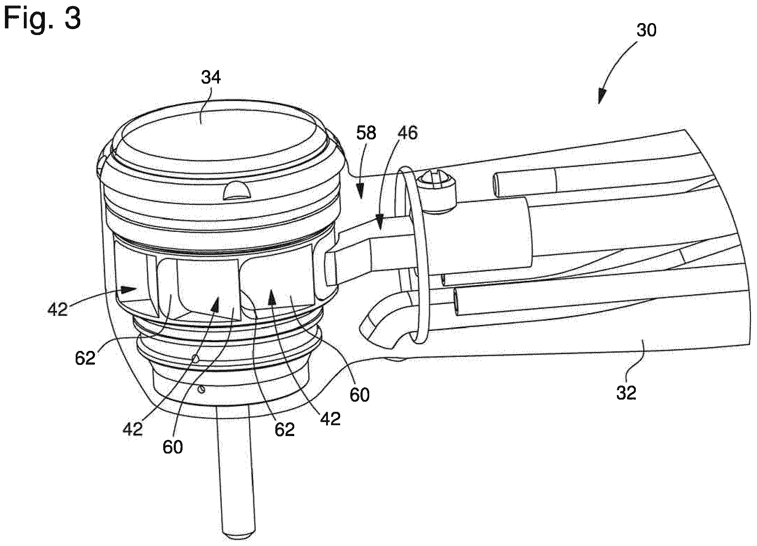

La figure 3 est une vue de côté, partiellement en transparence, de la tête 34 et du manche 32 de la pièce à main 30 à air comprimé selon l'invention. Comme on peut le voir à l'examen de cette figure, chacune des aubes 42 de la turbine 36 se compose d'une première surface 60 qui s'étend depuis le diamètre extérieur 44 de la roue mobile 38 et qui mène jusqu'à une seconde surface 62 qui s'étend radialement selon un rayon R de la roue mobile 38.

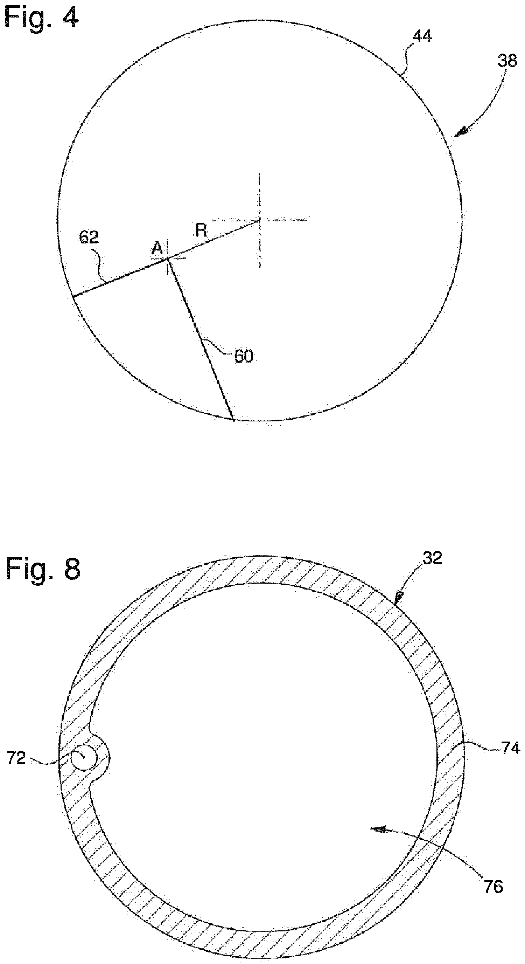

Plus précisément (voir également la figure 4), la première surface 60 s'étend depuis le diamètre extérieur 44 de la roue mobile 38 jusqu'à un point A du rayon R de la roue mobile 38 à partir duquel s'étend la seconde surface 62 jusqu'au diamètre extérieur 44 de la roue mobile 38. La première surface 60 est avantageusement plane, tandis que la seconde surface 62 présente un profil en arc de cercle tangent au rayon R de la roue mobile 38. Préférentiellement, la première surface 60 forme avec la seconde surface 62 un angle droit.

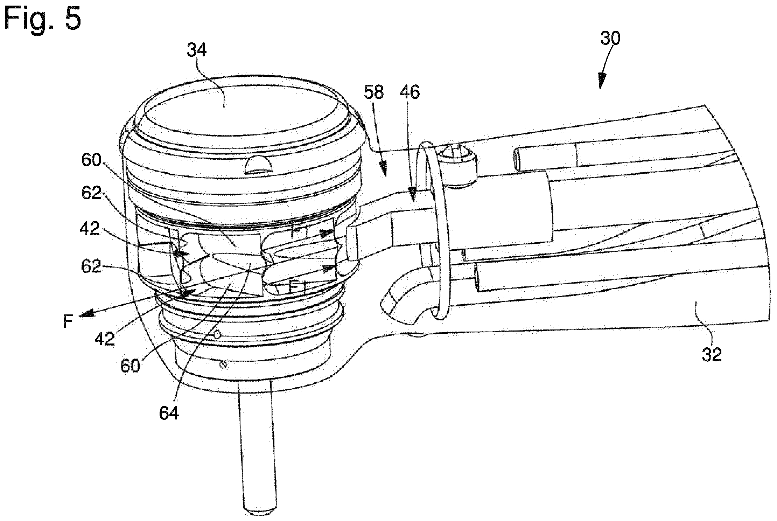

Enfin (voir figure 5), selon une variante préférée de réalisation de l'invention, les aubes 42 sont associées par paires de manière étagée, chaque aube 42 étant séparée de l'aube à laquelle elle est associée par une échancrure 64 qui s'étend dans la direction du flux d'air comprimé F, de sorte que le flux d'air F se partage en deux parts égales F1 au moment où il atteint l'échancrure 64. Cette échancrure 64 a pour but de réduire le temps de passage d'une aube à l'aube suivante afin de limiter les pertes de puissance mécanique de la turbine 36.

De ce qui précède, on comprend que la première surface 60 des aubes 42 conduit vers la seconde surface 62 l'air comprimé qui sort des moyens d'injection. Comme la seconde surface 62 s'étend selon un rayon R de la roue mobile 38 et que le flux d'air comprimé est canalisé selon une direction tangente au diamètre extérieur 44 de la roue mobile 38, le flux d'air comprimé tombe perpendiculairement sur la seconde surface 62 de l'aube 42 puis fait un demi-tour en étant défléchie par le profil en arc de cercle de la seconde surface 62. Or, la puissance mécanique délivrée par une turbine est d'autant plus grande que l'angle de déviation de l'air comprimé est important. Dans le cas de la présente invention, l'angle de déviation de l'air comprimé est de 180°, ce qui correspond à un optimum de conversion entre l'énergie pneumatique de l'air comprimé et l'énergie cinétique de la turbine 36.

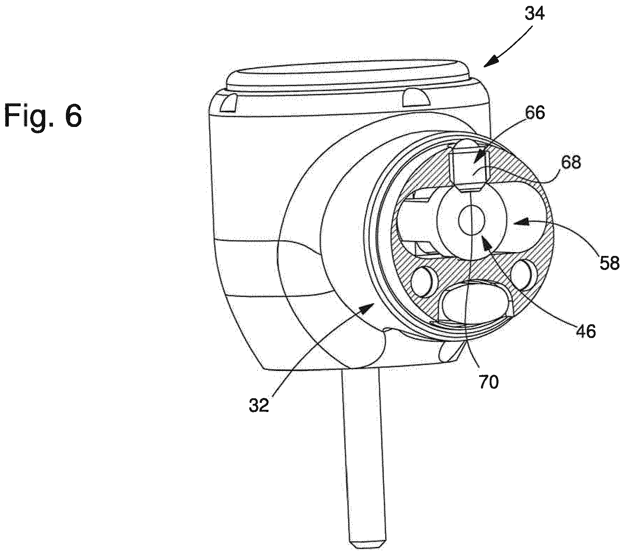

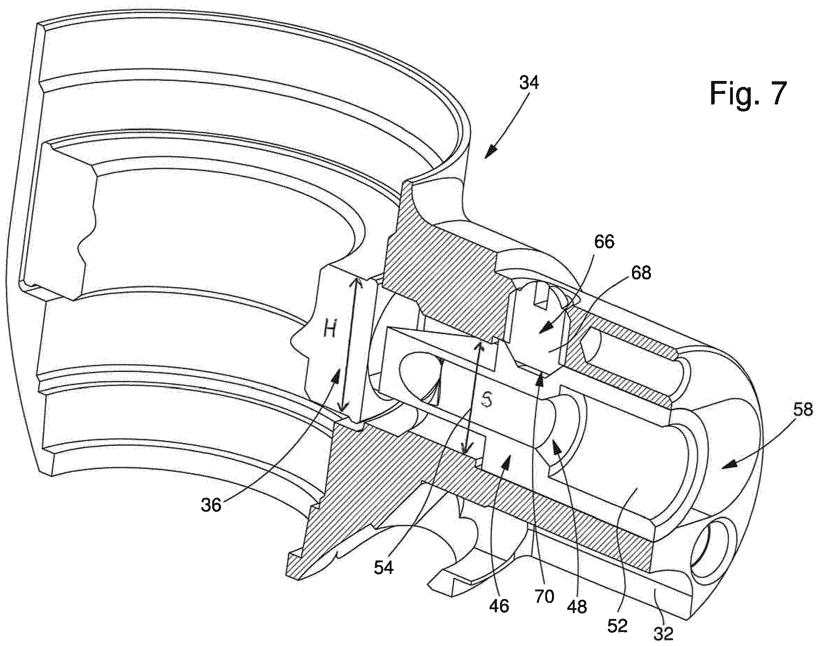

La figure 6 est une vue de l'arrière de la tête 34 de la pièce à main 30 à air comprimé selon l'invention, dans la zone où la tête 34 de la pièce à main 30 se raccorde au manche 32 de la pièce à main 30, et la figure 7 est une vue en coupe selon un plan vertical passant par l'axe longitudinal de symétrie de la tête 34 et d'une portion du manche 32 de la pièce à main 30 à air comprimé selon l'invention. Il ressort notamment clairement de ces deux figures que l'insert 46 qui sert à forcer le flux d'air comprimé en direction des aubes 42 de la roue mobile 38 est monté à l'intérieur du conduit d'échappement 58 ménagé dans le manche 32 de la pièce à main 30 et par lequel s'échappe l'air après avoir frappé les aubes 42 de la roue mobile 38. Préférentiellement, l'insert 46 est monté de manière coaxiale à l'intérieur du conduit d'échappement 58. De la sorte, le conduit d'échappement 58 est le plus grand possible et se trouve aussi près que possible du conduit 48 d'amenée du flux d'air comprimé, ce qui facilite le retour d'air et permet de maximiser la chute de pression, de sorte que la conversion entre l'énergie pneumatique de l'air comprimé et l'énergie cinétique de la turbine 36 est optimisée. On observe également à l'examen de la figure 7 que la section S du conduit d'échappement 58 s'étend sur au moins la hauteur H des aubes 42 de la roue mobile 38.

Selon encore une autre caractéristique de l'invention, l'insert 46 comprend des moyens pour le réglage de sa position à l'intérieur du manche 32 de la pièce à main 30. Selon un exemple donné à titre purement illustratif et non limitatif seulement, les moyens de réglage comprennent une vis de réglage 66 dont un pied 68 est reçu dans un siège 70 ménagé à la périphérie de l'insert 46. Le siège 70 pourrait être remplacé par deux plats.

Il va de soi que la présente invention n'est pas limitée au mode de réalisation qui vient d'être décrit, et que diverses modifications et variantes simples peuvent être envisagées par l'homme du métier sans sortir du cadre de l'invention tel que défini par les revendications annexées. En particulier, la présente invention enseigne que les moyens d'injection de l'air comprimé se présentent sous la forme d'un insert 46 indépendant monté à l'intérieur de la pièce à main 30 et à l'intérieur duquel est ménagé un conduit 48 d'amenée du flux d'air comprimé. Cette solution a pour but de surmonter les problèmes que rencontrerait l'homme du métier s'il cherchait à usiner de biais un conduit d'amenée de l'air comprimé par des techniques classiques dans le manche par exemple en acier inoxydable d'une pièce à main.

Selon une variante sortant du cadre de l'invention, les moyens 72 d'injection de l'air comprimé sont ménagés dans la paroi 74 du manche 32 de la pièce à main 30, et les moyens 76 d'échappement de l'air comprimé occupent la majeure partie de la section du manche 32. Un tel agencement peut typiquement être obtenu par les techniques dites d'impression en trois dimensions.

## Revendications

1. Pièce à main à air comprimé comprenant un manche (32) qui se raccorde à une tête (34) à l'intérieur de laquelle est logée une turbine (36), la turbine (36) comprenant une roue mobile (38) depuis une périphérie (40) de laquelle s'étendent une pluralité d'aubes (42) qui définissent un diamètre extérieur (44) de la roue mobile (38), la pièce à main (30) comprenant également des moyens d'injection ayant pour fonction de diriger sur les aubes (42) de la roue mobile (38) un flux d'air comprimé dont l'énergie pneumatique se transforme en énergie cinétique lorsque le flux d'air comprimé frappe les aubes (42) de la roue mobile (38), la pièce à main (30) comprenant de plus un conduit d'échappement (58) ménagé dans le manche (32) de la pièce à main (30) et par lequel s'échappe l'air après avoir frappé les aubes (42) de la roue mobile (38), le conduit d'échappement (58) communiquant avec la tête (34) via une ouverture qui se trouve au moins en regard de la zone dans laquelle l'air comprimé frappe les aubes (42) de la roue mobile (38),
les moyens d'injection de l'air comprimé s'étendant à l'intérieur du conduit d'échappement (58), cette ouverture entourant complètement les moyens d'injection de l'air comprimé,
**caractérisée en ce que**
les moyens d'injection de l'air comprimé se présentent sous la forme d'un insert (46) à l'intérieur duquel est ménagé un conduit (48) d'amenée du flux d'air comprimé et qui est rapporté à l'intérieur du manche (32) de la pièce à main (30),
l'insert (46) étant disposé à l'intérieur du conduit d'échappement (58), l'insert (46) étant monté de manière coaxiale à l'intérieur du conduit d'échappement (58),
la section du conduit d'échappement (58) s'étendant sur au moins la hauteur des aubes (42) de la roue mobile (38).

2. Pièce à main à air comprimé selon la revendication précédente, **caractérisée en ce que** l'insert (46) est agencé de façon que l'axe central (56) du flux d'air comprimé (F) s'étende selon une direction tangentielle au diamètre extérieur (44) de la roue mobile (38).

3. Pièce à main à air comprimé selon l'une quelconque des revendications 4 précédentes, **caractérisée en ce que** le conduit (48) d'amenée du flux d'air comprimé se compose d'un premier et d'un second conduit rectilignes (52, 54) disposés dans le prolongement l'un de l'autre, le second conduit rectiligne (54) présentant un axe longitudinal de symétrie (56) qui s'étend selon une direction tangentielle au diamètre extérieur (44) de la roue mobile (38).

4. Pièce à main à air comprimé selon l'une quelconque des revendications 4 précédentes, **caractérisée en ce que** l'insert (46) comprend des moyens pour le réglage de sa position à l'intérieur du manche (32) de la pièce à main (30).

5. Pièce à main à air comprimé selon la revendication 4, **caractérisée en ce que** les moyens de réglage comprennent soit un siège (70) pour recevoir une vis de réglage (66), soit deux plats.

6. Pièce à main à air comprimé selon l'une quelconque des revendications précédentes, **caractérisée en ce que** chacune des aubes (42) de la turbine (36) se compose d'une première surface (60) qui s'étend depuis le diamètre extérieur (44) de la roue mobile (38) et qui mène jusqu'à une seconde surface (62) qui s'étend selon un rayon (R) de la roue mobile (38).

7. Pièce à main à air comprimé selon la revendication 6, **caractérisée en ce que** la première surface (60) s'étend depuis le diamètre extérieur (44) de la roue mobile (38) jusqu'à un point (A) du rayon (R) de la roue mobile (38) à partir duquel s'étend la seconde surface (62) jusqu'au diamètre extérieur (44) de la roue mobile (38).

8. Pièce à main à air comprimé selon la revendication 7, **caractérisée en ce que** la première surface (60) est plane, tandis que la seconde surface (62) présente un profil en arc de cercle tangent au rayon (R) de la roue mobile (38).

9. Pièce à main à air comprimé selon l'une quelconque des revendications 6 à 8, **caractérisée en ce que** les aubes (42) sont associées par paires de manière étagée, chaque aube (42) étant séparée de l'aube à laquelle elle est associée par une échancrure (64) qui s'étend dans la direction du flux d'air comprimé (F), de sorte que le flux d'air comprimé (F) se partage en deux parts égales (F1) au moment où il atteint l'échancrure (64).

## Patentansprüche

1. Drucklufthandstück umfassend einen Griff (32), der sich mit einem Kopf (34) verbindet, in dessen Innerem eine Turbine (36) angeordnet ist, wobei die Turbine (36) ein Laufrad (38) umfasst, von dessen Umfang (40) sich eine Mehrzahl von Radschaufeln (42) erstrecken, die einen Außendurchmesser (44) des Laufrades (38) definieren, wobei das Handstück (30) ferner Einblasmittel umfasst, die die Funktion haben, einen Druckluftfluss auf die Schaufeln (42) des Laufrades (38) zu lenken, dessen pneumatische Energie sich in kinetische Energie umwandelt, wenn der Druckluftfluss auf die Schaufeln (42) des Laufrades (38) auftrifft, wobei das Handstück (30) ferner eine Auslassleitung (58) umfasst, die im Griff (32) des Handstücks (30) angeordnet ist und durch die die Luft austritt, nachdem sie auf die Schaufeln (42) des Laufrades (38) aufgetroffen ist, wobei die Auslassleitung (58) mit dem Kopf (34) über eine Öffnung in Verbindung steht, die sich zumindest gegenüber dem Bereich befindet, in dem die Druckluft auf die Schaufeln (42) des Laufrades (38) auftrifft,
wobei die Mittel zum Einblasen der Druckluft sich im Inneren der Auslassleitung (58) erstrecken, wobei diese Öffnung vollständig die Mittel zum Einblasen der Druckluft umgibt,
**dadurch gekennzeichnet, dass**
die Mittel zum Einblasen der Druckluft in Form eines Einsatzes (46) ausgebildet sind, in dessen Innerem eine Leitung (48) zum Zuführen des Druckluftflusses angeordnet ist, und der an das Innere des Griffs (32) des Handstücks (30) angefügt ist,
wobei der Einsatz (46) im Inneren der Auslassleitung (58) angeordnet ist, wobei der Einsatz (46) in koaxialer Weise im Inneren der Auslassleitung (58) montiert ist,
wobei sich der Querschnitt der Auslassleitung (58) mindestens bis auf die Höhe der Schaufeln (42) des Laufrades (38) erstreckt.

2. Drucklufthandstück nach dem vorangehenden Anspruch, **dadurch gekennzeichnet, dass** der Einsatz (46) derart angeordnet ist, dass die Mittelachse (56) des Druckluftflusses (F) sich in einer zum Außendurchmesser (44) des Laufrades (38) tangentialen Richtung erstreckt.

3. Drucklufthandstück nach einem der vorangehenden Ansprüche, **dadurch gekennzeichnet, dass** die Leitung (48) zum Zuführen des Druckluftflusses aus einer ersten und einer zweiten geradlinigen Leitung (52, 54) besteht, die in der Verlängerung zueinander angeordnet sind, wobei die zweite geradlinige Leitung (54) eine Symmetrielängsachse (56) aufweist, die sich in einer zum Außendurchmesser (44) des Laufrades (38) tangentialen Richtung erstreckt.

4. Drucklufthandstück nach einem der vorangehenden Ansprüche, **dadurch gekennzeichnet, dass** der Einsatz (46) Mittel für die Regulierung seiner Position im Inneren des Griffs (32) des Handstücks (30) umfasst.

5. Drucklufthandstück nach Anspruch 4, **dadurch gekennzeichnet, dass** die Regulierungsmittel entweder einen Sitz (70) zum Aufnehmen einer Stellschraube (66) oder zwei Flachstellen umfassen.

6. Drucklufthandstück nach einem der vorangehenden Ansprüche, **dadurch gekennzeichnet, dass** jede der Schaufeln (42) der Turbine (36) aus einer ersten Oberfläche (60) besteht, die sich vom Außendurchmesser (44) des Laufrades (38) aus erstreckt und die bis zu einer zweiten Oberfläche (62) führt, die sich entlang einem Radius (R) des Laufrades (38) erstreckt.

7. Drucklufthandstück nach Anspruch 6, **dadurch gekennzeichnet, dass** die erste Oberfläche (60) sich vom Außendurchmesser (44) des Laufrades (38) bis zu einem Punkt (A) des Radius (R) des Laufrades (38) erstreckt, von dem aus sich die zweite Oberfläche (62) bis zum Außendurchmesser (44) des Laufrades (38) erstreckt.

8. Drucklufthandstück nach Anspruch 7, **dadurch gekennzeichnet, dass** die erste Oberfläche (60) eben ist, während die zweite Oberfläche (62) ein Kreisbogenprofil tangential zum Radius (R) des Laufrades (38) aufweist.

9. Drucklufthandstück nach einem der Ansprüche 6 bis 8, **dadurch gekennzeichnet, dass** die Schaufeln (42) paarweise stufenartig verbunden sind, wobei jede Schaufel (42) von der Schaufel, mit der sie verbunden ist, durch eine Einbuchtung (64) getrennt ist, die sich in der Richtung des Druckluftflusses (F) erstreckt, so dass der Druckluftfluss (F) sich in zwei gleiche Teile (F1) in dem Moment aufteilt, in dem er die Einbuchtung (64) erreicht.

## Claims

1. Compressed air handpiece including a handle (32), which is connected to a head (34), which houses a turbine (36), the turbine (36) including a moving wheel (38) from a periphery (40) of which extends a plurality of blades (42) which define an outer diameter (44) of the moving wheel (38), the handpiece (30) also including injection means, whose function is to direct onto the blades (42) of the moving wheel (38) a compressed air flow whose pneumatic energy is converted into kinetic energy when the compressed air flow strikes the blades (42) of the moving wheel (38), the handpiece (30) further including a discharge pipe (58), arranged in the handle (32) of the handpiece (30) and through which the air is discharged after striking the blades (42) of the moving wheel (38), the discharge pipe (58) communicating with the head (34) via an opening located at least opposite the area in which the compressed air strikes the blades (42) of the moving wheel (38), the compressed air injection means extending inside the discharge pipe (58), said opening completely surrounding the compressed air injection means, **characterized in that** the compressed air injection means take the form of an insert (46) inside which is arranged a compressed air flow feed pipe (48) and which is placed inside the handle (32) of the handpiece (30), the insert (46) being arranged inside the discharge pipe (58), the insert (46) being mounted coaxially inside the discharge pipe (58), the section of the discharge pipe (58) extending over at least the height of the blades (42) of the moving wheel (38).

2. Compressed air handpiece according to the preceding claim, **characterized in that** the insert (46) is arranged so that the central axis (56) of the compressed air flow (F) extends in a tangential direction to the outer diameter (44) of the moving wheel (38).

3. Compressed air handpiece according to any of the preceding claims, **characterized in that** the compressed air flow feed pipe (48) is formed of a first and a second rectilinear pipe (52, 54) arranged in the extension of each other, the second rectilinear pipe (54) having a longitudinal axis of symmetry (56) which extends in a tangential direction to the outer diameter (44) of the moving wheel (38).

4. Compressed air handpiece according to any of the preceding claims, **characterized in that** the insert (46) includes means of adjusting the position of the insert inside the handle (32) of the handpiece (30).

5. Compressed air handpiece according to claim 4, **characterized in that** the adjustment means include either a seat (70) for receiving an adjusting screw (66) or two flat portions.

6. Compressed air handpiece according to any of the preceding claims, **characterized in that** each of the blades (42) of the turbine (36) is formed of a first surface (60) which extends from the outer diameter (44) of the moving wheel (38) and which leads to a second surface (62) which extends along a radius (R) of the moving wheel (38).

7. Compressed air handpiece according to claim 6, **characterized in that** the first surface (60) extends from the outer diameter (44) of the moving wheel (38) to a point (A) on the radius (R) of the moving wheel (38) from which the second surface (62) extends to the outer diameter (44) of the moving wheel (38).

8. Compressed air handpiece according to claim 7, **characterized in that** the first surface (60) is plane, whereas the second surface (62) has a profile in the shape of an arc of a circle tangential to the radius (R) of the moving wheel (38).

9. Compressed air handpiece according to any of claims 6 to 8, **characterized in that** the blades (42) are associated in pairs in a stepped manner, each blade (42) being separated from the blade associated therewith by a notch (64) which extends in the direction of the compressed air flow (F), so that the compressed air flow (F) is divided into two equal parts (F1) at the moment when the compressed air flow reaches the notch (64).
